# EUROPEAN PATENT APPLICATION

(11) **EP 1 743 586 A1**
(43) Date of publication of application: **17.01.2007**
(21) Application number: 06117274.8
(22) Date of filing: 14.07.2006
(51) Int. Cl.: A61B 17/80

(54) **Clavicular bone plate**

(30) Priority: 14.07.2005 CA 2512229
(71) Applicant: Terray Corporation, Arnprior, Ontario K7S 3GB (CA)
(72) Inventor: Feibel, Robert, Arnprior Ontario K7S 3G8 (CA); Calder, Patricia, Arnprior Ontario K7S 3GB (CA)
(74) Representative: Bailey, David Martin

(57) **Abstract**

A bone plate (1) for use in setting a clavicle, comprising an elongate portion (2) and an arch portion (3), the elongate portion having a curve approximately the shape of the anterior surface of the clavicle, the arch portion being adapted to fit over a clavicle in the region of the acromial end thereof.

## Description

The present invention relates to the field of implantable orthopaedic devices. In particular, the present invention provides a bone plate configured for use with a human clavicle.

The clavicle is a long bone, composed primarily of cancellous and cortical tissue, that forms the anterior portion of the shoulder girdle. It articulates at its medial end with the manubrium sterni, and at its lateral end with the acromion of the scapula.

Because of its key position in the shoulder girdle, a large portion of the surface of the clavicle is devoted to attachment sites for a variety of muscles and ligaments. For instance, the Deltoideus and Trapezius attach to the upper surface, and the under surface gives rise to the conoid and trapezoid ligaments. The anterior border has attachment surfaces for the Pectoralis major and Deltoideus. The superior border has an attachment surface for the Sternocleidomastoideus. The posterior border attaches to the Subclavius and to cervical fascia of the Omohyoideus.

The anterior surface has an attachment for the Pectoralis major, and the Sternohyoideus, as well as the costoclavicular ligament, the Subclavius and the coracoclavicular fascia.

The inferior surface attaches to the costoclavicular ligament, the Subclavius, the coracoclavicular fascia, and the intermuscular septum of the Subclavius.

The sternal extremity attaches to numerous ligaments, and the acromial extremity attaches to the acromioclavicular ligaments.

It will be appreciated, in view of the foregoing, that a fracture of the clavicle can be an extremely serious injury, and one which has been difficult to treat. That is, because it attaches to so many muscles and ligaments, the clavicle is, during any period of activity, subject to stresses in many directions. It is also subject to significant torque, primarily because of its articulation with the scapula, and attachment to muscles that attach to the scapula and sternum. Accordingly, if the clavicle suffers a minor fracture, the forces acting on it may tend to exacerbate the fracture. If a major fracture is suffered, the acromial fragment especially may be difficult to re-align with the sternal fragment. Furthermore, because the clavicle is embedded within the upper part of the thorax, rather than being part of a limb, it is extremely difficult to set the clavicle with an external splint or cast.

However, because so much of the surface of the clavicle is covered with muscle and ligament attachment sites, it is also extremely difficult to position and attach a conventional bone plate to the surface. Moreover, a bone plate on the surface may not be effective against torquing of the clavicle.

There exists a specialized type of bone plate for use with ribs, shown in U.S. Patent Publication No. 2005/085819 (Ellis et al.) that may, in some circumstances, be used effectively to set clavicular fractures. That bone plate includes a flat plate portion provided with screw holes for attachment to a bone surface, and clip portions that may or may not be integral with the flat plate portions, to clip over a rib bone. The clip portions have apertures configured to receive a screw passing through the rib bone, and lock the screw in place, thereby firmly anchoring the plate on the rib.

For a small number of clavicle fractures, the Ellis et al. plate has applicability. However, the Ellis et al. plate is not correctly shaped to be fastened to a clavicle, in that it is a straight plate with an orthogonally extending clip portion. Accordingly the axis of the plate portion of the Ellis et al. implant will not follow the axis of the clavicle, nor is it shaped to avoid muscle and ligament attachment sites on the clavicle.

The bone plate of the present invention overcomes deficiencies of the known prior art by providing a clavicle bone plate shaped to be fastened to the anterior and superior surfaces of a clavicle with minimal disruption of the muscle and ligament attachment surfaces.

The clavicle plate of the present invention has a curved longitudinal axis, to conform with the axis of the clavicle. Screw holes on the clavicle plate of the present invention are positioned to inflict minimal damage to the clavicle, while ensuring a strong fit with those fragments of the clavicle that are being set with the plate by the present invention. Screw holes are provided on the anterior and superior surfaces of the plate of the present invention, whereby the plate of the present invention will resist both torquing and bending of a clavicle during healing.

In a broad aspect, then, the present invention relates to a bone plate for use in setting a clavicle, comprising an elongate portion and an arch portion, said elongate portion having a curve approximately the shape of the superior surface of the clavicle, said arch portion being adapted to fit over a clavicle in the region of the acromial end thereof.

The arch portion may comprise a substantially flat inferior element, an arched anterior element, and a substantially flat superior element integral with said elongate portion.

Preferably the sternal extremity of said elongate portion is curved in an anterior direction.

Moreover, the elongate portion will be provided with a series of screw holes along its length.

In a preferred embodiment, the screw holes along said elongate portion are oblong, with a ramped lower surface, to compress a fracture as a screw is tightened.

Furthermore, the arch portion may have screw holes formed therein, on at least the anterior and superior surfaces thereof.

The screw hole on the anterior surface of said arch portion may be tilted so as to guide a screw into said clavicle at an angle to said arch portion.

The above and other aspects of the present invention will now be described in further detail with reference, by way of example only, to the accompanying drawings, in which:
Figure 1 is a superior view of an embodiment of a clavicle plate according to the present invention;
Figure 2 is an inferior view of the clavicle plate shown in Figure 1;
Figure 3 is a distal or acromial view of an embodiment of a clavicle plate according to the present invention;
Figure 4 is a cross-sectional view through line 4-4 of Figure 2;
Figure 5 is a cross-sectional view through line 5-5 of Figure 4;
Figure 6 is a cross-sectional view through line 6-6 of Figure 1;
Figure 7 is a superior-anterior perspective view of an embodiment of a clavicle plate according to the present invention;
Figure 8 is an inferior-posterior perspective view of an embodiment of a clavicle plate according to the present invention.

Referring now to the drawings, a clavicle plate 1 for setting of fractured clavicles is shown. The clavicle plate 1 includes two distinct parts, namely a longitudinal plate portion 2 that is designed for attachment to the superior surface of a clavicle, and an arch portion 3 at the lateral or acromial end of the longitudinal portion.

The longitudinal plate surface is shaped with a slight curve that approximates the curves in the anterior and superior surfaces of the clavicle, to permit the plate to be fastened to the superior surface of a clavicle with minimal disruption of the muscle and ligament attachment function of the clavicle. The longitudinal portion includes a series of screw holes 4 formed therein to permit it to be screwed to the superior surface of the clavicle.

The arch portion, that extends over the clavicle at the acromial extremity of the plate, also includes screw holes, one or two (two illustrated) in the portion of the arch substantially coplanar with the longitudinal portion and another screw hole in the front portion of the arch, for placement of a screw in the anterior surface of the clavicle.

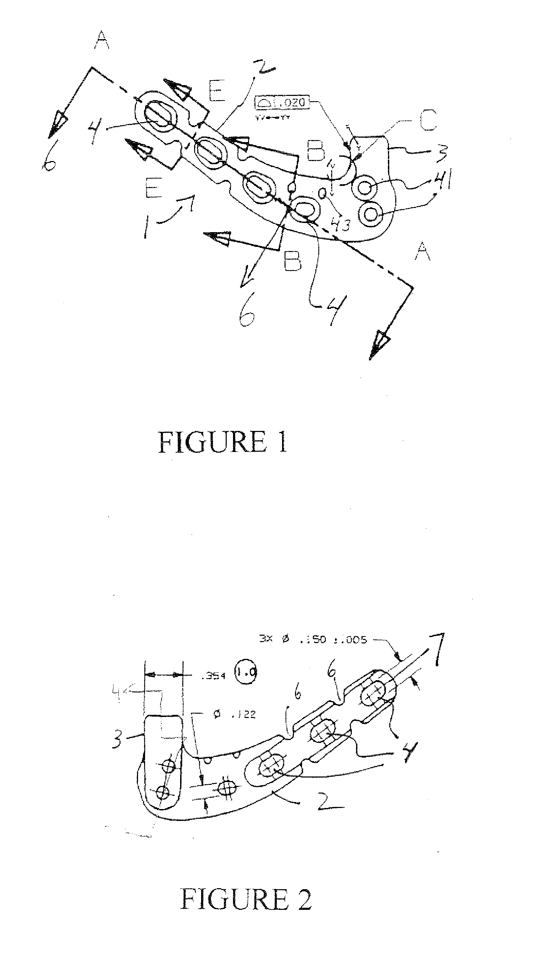

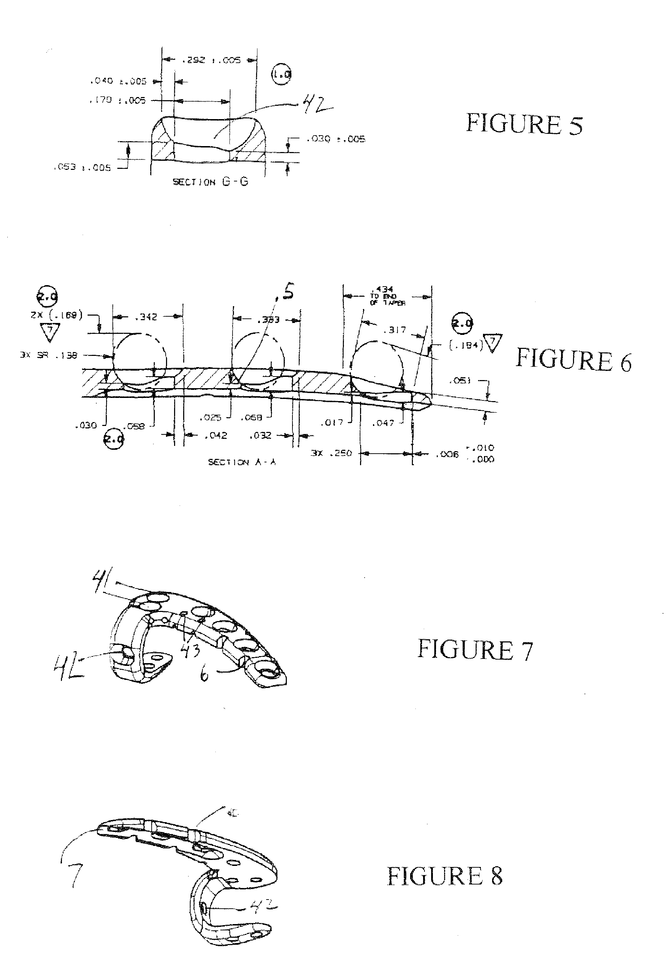

The longitudinal portion, as seen in Figures 1 and 6, has screw holes along its length. Each screw hole is a conventionally shaped oblong screw hole, with a countersink biased such that when a screw is driven in, tightening the screw will tend to pull the bone to a straight and compressed condition. This is seen most clearly in Figure 6, wherein wedge-like surfaces 5 around the sternal side of the screw holes are seen. As a screw being driven into such a hole is tightened, the screw head will bear against the wedge-like edge of the screw hole, and tend to slide down the wedge, thereby pulling the distal or acromial fragment toward the sternal fragments of the broken clavicle. This results in optimal reduction and compression of the fracture site to ensure interfragment contact for optimal healing.

Notches 6 on each side edge of the longitudinal portion of the bone plate are provided to facilitate custom contouring of the plate by a surgeon during the implantation procedure. The notches establish natural bending points between the screw holes to lessen the likelihood of deformation in the region of a screw hole when the bone plate is bent by a surgeon.

A channel 7 is formed in the undersurface of the longitudinal portion. This channel provides a pair of relatively parallel edges that can grip the surface of the bone with minimized contact when the plate is tightened against the bone.

At the acromial end of the plate, the arch portion 3 has a pair of screw holes 41 on its superior face that are set at an angle to the principle axis of the arch. There are a pair of aligned holes on the inferior surface of the arch, to accommodate the end of a screw in order to reduce the possibility of damage to the distal fragment through screw toggle. Capturing the screws on the underside of the arm has the added advantage of relying on the plate and screw interface to resist torques across the acromioclavicular joint rather than the fragment the surgeon is attempting to immobilize. The presence of holes on the opposite arch makes the use of wire to affix the arch portion to the clavicle an option.

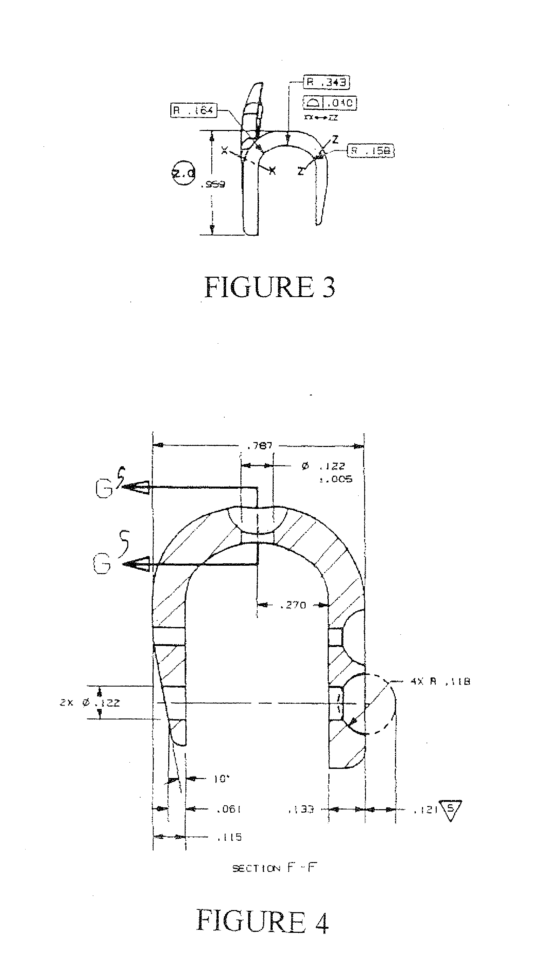

There is also a screw hole 42 on the anterior surface of the arch, as can be seen in Figure 4 and Figure 7. As shown in detail in Figure 5, this screw hole is slightly tilted to direct a screw posteriorly or at a slight angle toward the sternal end of the plate and across the fracture site. This feature, in combination with the angle at which the screw holes are aligned at the superior surface of the arch, will prevent a screw driven into the anterior surface of the clavicle, through the front screw hole, from striking either of the screws in the top of the arch.

A pair of additional holes 43 are provided on the superior-anterior surface of the plate, and may accommodate sutures, if sutures are required for the reattachment of ligaments.

It will be observed that the use of a robust arch portion on the acromial end of the plate will counter any tendency of the healing clavicle to torque. The use of a longitudinal, or medial portion, that is curved to closely resemble the anatomical curvature of a clavicle provides the greatest probability of reaching and immobilizing additional medial fragments of the fractured clavicle.

## Claims

1. A bone plate for use in setting a clavicle, comprising an elongate portion and an arch portion, said elongate portion having a curve approximately the shape of the superior surface of the clavicle, said arch portion being adapted to fit over a clavicle in the region of the accromial end thereof.

2. A bone plate as claimed in claim 1, wherein said arch portion comprises a substantially flat inferior element, an arched anterior element, and a substantially flat superior element integral with said elongate portion.

3. A bone plate as claimed in claim 1 or claim 2, wherein the sternal extremity of said elongate portion is curved in an anterior direction.

4. A bone plate as claimed in any preceding claim, wherein said elongate portion is provided with a series of screw holes along its length.

5. A bone plate as claimed in claim 4, wherein the screw holes along said elongate portion are oblong, with a ramped lower surface, to compress a fracture as a screw is tightened.

6. A bone plate as claimed in any preceding claim, wherein said arch portion has screw holes formed therein, on at least the anterior and superior surfaces thereof.

7. A bone plate as claimed in claim 6, wherein the screw hole on the anterior surface of said arch portion is tilted so as to guide a screw into said clavicle at an angle to said arch portion.
